# EUROPÄISCHE PATENTSCHRIFT

(11) **EP 3 289 096 B1**
(45) Veröffentlichungstag und Bekanntmachung des Hinweises auf die Patenterteilung: **21.07.2021**
(21) Anmeldenummer: 16720099.7
(22) Anmeldetag: 28.04.2016
(51) Int. Cl.: C12Q 1/6883

(54) **VERFAHREN ZUR PROGNOSE UND/ODER DIAGNOSE EINER KRANKHEIT AUF BASIS VON EINER PROBE AUS FETTGEWEBE**
METHOD FOR THE PROGNOSIS AND/OR DIAGNOSIS OF AN ILLNESS BASED ON A SAMPLE OF ADIPOSE TISSUE
MÉTHODE POUR EFFECTUER LE PRONOSTIC ET/OU LE DIAGNOSTIC D'UNE MALADIE SUR LA BASE D'UN ÉCHANTILLON DE TISSUS ADIPEUX

(30) Priorität: 30.04.2015 DE 102015208083
(43) Veröffentlichungstag der Anmeldung: 07.03.2018
(73) Patentinhaber: Lipozyt Marker GmbH, 28357 Bremen (DE)
(72) Erfinder: THIES, Helge Wilhelm, 28359 Bremen (DE); KLEMKE, Markus, 28777 Bremen (DE)
(74) Vertreter: Eisenführ Speiser
(86) Internationale Anmeldenummer: PCT/EP2016/059556
(87) Internationale Veröffentlichungsnummer: WO 2016/174169

(56) Entgegenhaltungen:
- WO-A1-2014/006163
- TATIANA A. LARKINA ET AL: "HMG1A and PPARG are differently expressed in the liver of fat and lean broilers", JOURNAL OF APPLIED GENETICS: AN INTERNATIONAL JOURNAL OF GENETICS AND BREEDING, Bd. 52, Nr. 2, 1. Mai 2011 (2011-05-01), Seiten 225-228, XP055283304, Germany ISSN: 1234-1983, DOI: 10.1007/s13353-010-0023-z
- HELGE W. THIES ET AL: "Permanent activation of HMGA2 in lipomas mimics its temporal physiological activation linked to the gain of adipose tissue", OBESITY RESEARCH, Bd. 22, Nr. 1, 17. Januar 2014 (2014-01-17), Seiten 141-150, XP055283303, US ISSN: 1930-7381, DOI: 10.1002/oby.20137
- VERNOCHET C ET AL: "PPARgamma-dependent and PPARgamma-independent effects on the development of adipose cells from embryonic stem cells", FEBS LETTERS, ELSEVIER, AMSTERDAM, NL, Bd. 510, Nr. 1-2, 2. Januar 2002 (2002-01-02), Seiten 94-98, XP027305780, ISSN: 0014-5793 [gefunden am 2002-01-02] in der Anmeldung erwähnt
- CHRISTIANE WINKLER ET AL: "Lack of Association of Type 2 Diabetes Susceptibility Genotypes and Body Weight on the Development of Islet Autoimmunity and Type 1 Diabetes", PLOS ONE, Bd. 7, Nr. 4, 25. April 2012 (2012-04-25), Seite e35410, XP055283298, DOI: 10.1371/journal.pone.0035410

## Beschreibung

Die vorliegende Erfindung betrifft ein Verfahren zur Prognose von Adipositas sowie zur Prognose und/oder Diagnose von Diabetes basierend auf einer Probe aus Fettgewebe, wobei eine Eingruppierung des Spenders in eine von wenigstens vier Risikogruppen erfolgt.

Die zunehmende Verbreitung von Übergewicht und Adipositas stellt weltweit ein wachsendes Gesundheitsproblem dar. Vor allem Adipositas ist assoziiert mit Herz-Kreislauf-Krankheiten (CVD), einem erhöhten Risiko für Diabetes mellitus Typ II und/oder der Entwicklung des Metabolischen Syndroms. Definiert werden Übergewicht und Adipositas über den Body-Mass-Index (BMI), dieser beschreibt das Verhältnis des Körpergewichts zur Körpergröße. Der BMI wird nach der folgenden Formel bestimmt: BMI = Körpergewicht [kg] / Körpergröße zum Quadrat [m²]. Laut WHO-Kriterien liegt der Normal-BMI zwischen 18,5 und 24,9 kg/m², Übergewicht liegt bei einem BMI von über 25 kg/m² vor, während ab einem BMI von 30 kg/m² von Adipositas gesprochen wird. Adipositas wird in weitere Grade aufgeteilt: Adipositas Grad I (BMI = 30-34,9), Adipositas Grad II (BMI= 35-39,9), Adipositas Grad III (BMI ≥ 40) sowie Super-Adipositas (BMI ≥ 50). Ein hoher BMI sagt aber wenig über die eigentliche Körpergewebszusammensetzung aus und ist nur ein erster grober Indikator in Hinblick auf die Möglichkeit, an Adipositas-assoziierten Krankheiten zu erkranken. Neuere Studien zeigen, dass Individuen mit einem hohen BMI (BMI ≥ 30 kg/m²) nicht zwangsläufig an Herz-Kreislauf-Krankheiten und Diabetes mellitus Typ II leiden müssen (sogenannte "Metabolically healthy Obese"), wohingegen Individuen mit einem normalen BMI (18,5 - 24,9 kg/m²) trotz ihres niedrigen BMIs an den genannten Erkrankungen leiden können.

Ursächlich für die Entstehung von Übergewicht und Adipositas sind Wachstumsdynamiken im Fettgewebe, die ausgelöst werden, wenn die Energieaufnahme durch die Nahrung den Energieverbrauch über längere Zeit übersteigt. Hierbei wird überschüssige Energie in Form von Triglyzeriden im Fettgewebe gespeichert. Die Zunahme von Fettgewebe im Körper, die zu Übergewicht und Adipositas führen kann, basiert sowohl auf dem Anstieg der Anzahl an Adipozyten (Hyperplasie) als auch auf der Zunahme des Volumens der Adipozyten (Hypertrophie). Die Hypertrophie wird hierbei als initiales Merkmal des beginnenden Übergewichts und zukünftig möglicher Adipositas gesehen. Da die Adipozyten nur in einem begrenzten Maße wachsen und Triglyzeride speichern können, ist die Neubildung von Adipozyten im Falle zu hoher Energieaufnahme zwingend erforderlich. Allerdings kann die Zunahme der Zahl von Adipozyten sowohl im Fall von entstehendem oder zunehmendem Übergewicht als auch bei der normalen Bildung von Fettgewebe beobachtet werden.

Während der Adipogenese differenziert sich der fibroblastenähnliche Präadipozyt zum lipidspeichernden, Insulin-sensitiven reifen Adipozyten. Dieser Differenzierungsvorgang ist äußerst dynamisch und verläuft über mehrere Entwicklungsstufen, an denen diverse Transkriptionsfaktoren beteiligt sind. Der Ausgangspunkt der Adipogenese ist die mesenchymale Stammzelle (MSC), welche durch Selbsterneuerung in der Lage ist, den Stammzell-Pool konstant zu halten und andererseits nach einem entsprechenden Stimulus in diverse Gewebetypen differenzieren kann. Tang et al. (Tang W, Zeve D, Suh JM, Bosnakovski D, Kyba M, Hammer RE, Tallquist MD, Graff JM (2008) White fat progenitor cells reside in the adipose vasculature. Science 322:583-6) konnten diese Vorläuferzellen in der perivaskulären Nische des Fettgewebes nachweisen. Ist die MSC zum determinierten Präadipozyten differenziert, folgt eine Phase der Zellteilung bis zu einem Punkt, an dem die Präadipozyten durch Kontaktinhibition ihr Wachstum einstellen und im Zellzyklus arretiert werden. Nach einem hormonellen Stimulus treten die wachstumsarretierten Präadipozyten erneut in den Zellzyklus ein und durchlaufen eine Phase der verstärkten Proliferation, die als mitotische klonale Expansion bezeichnet wird. Nach der klonalen Expansion folgt ein erneuter Zellzyklusarrest der Präadipozyten und die terminale Differenzierung beginnt mit der transkriptionalen Aktivierung von bestimmten Adipozyten-spezifischen Genen. Eine Kaskade von Transkriptionsfaktoren wird schrittweise aktiviert und führt zum Insulin-sensitiven reifen Adipozyten.

Bislang gibt es nur ungenaue und indirekte Marker wie etwa abdominales Übergewicht, Bluthochdruck und erhöhte Blutglukosewerte usw., um das Risiko abzuschätzen, zukünftig an einer Adipositas oder an einer der folgenden Adipositas-assoziierten Krankheiten zu erkranken: Metabolisches Syndrom, CVD und Diabetes mellitus Typ II. Verlässliche und aussagekräftige prädiktive Marker existieren für die genannten Erkrankungen bis heute nicht. Obwohl Übergewicht einen wichtigen Risikofaktor für diese Erkrankungen darstellt, ist allgemein anerkannt, dass Körperfettgewebs-Verteilung und eine Anhäufung von unreifem Fettgewebe einen zusätzlichen, unabhängigen Faktor repräsentieren. Es ist bekannt, dass Dysfunktionen während der Adipogenese zu einer Anhäufung unreifer Fettzellen führen, wodurch langfristig die Bildung einer Diabetes-Erkrankung (Diabetes mellitus Typ II) und des Metabolischen Syndroms begünstigt wird. Bis heute gibt es kein zuverlässiges Diagnoseverfahren, um auf molekularer Ebene unreifes Fettgewebe zu identifizieren.

Die Punktion des subkutanen Fettgewebes (WAT) ist eine einfache, sichere und wenig invasive Methode, um Fettgewebe zu erhalten, doch enthalten die Aspirate zu wenig Zellen, um eine zuverlässige molekulare Analyse dieser Zellen zu gewährleisten. Besonders im WAT schwach exprimierte Gene können nicht immer zuverlässig detektiert werden. Nachfolgend werden Biomarker und Methoden beschrieben, die die Identifizierung von Individuen mit einem unreifen Fettgewebe ermöglichen, basierend u.a. auf Quantifizierungen der Genexpression in Proben von WAT.

Das HMGA2-Protein spielt eine bedeutende Rolle in einer Vielzahl von biologischen Prozessen wie Wachstum, Proliferation und Differenzierung. Rearrangierungen der Chromosomenregion 12q13∼15, die mit einer Aktivierung der sonst inaktivierten *HMGA2-Allele* assoziiert sind, führen häufig zu Lipomen, gutartigen Tumoren der Fettgewebszellen. In Hinblick auf normale Zellen konnten Studien den bedeutenden Einfluss von *HMGA2* während des Wachstums und der Entwicklung von Adipozyten belegen. Die EP 2 682 752 A1 offenbart, dass auf Basis der Expressionslevel von *HMGA2* eine Prognose und/oder Diagnose von Diabetes erfolgen kann. Ferner weist dieses Dokument darauf hin, dass die *HMGA2-*Expressionslevel und die PPAR-gamma-Expressionslevel sich zueinander reziprok verhalten. Die Forschungen der Anmelder haben jedoch ergeben, dass das in diesem Dokument offenbarte Risikoeingruppierungsmuster zu grob ist.

Daher war es Aufgabe der vorliegenden Erfindung, ausgehend von dem Stand der Technik, insbesondere der EP 2 682 752 A1 ein verbessertes Prognose- und/oder Diagnoseverfahren anzugeben. Diese Aufgabe wird gelöst durch ein Verfahren zur Prognose einer Adipositas und zur Prognose und/oder Diagnose von Diabetes, umfassend die Schritte:
a) Bereitstellen einer Probe aus Fettgewebe aus einem Spender,
b) Bestimmen des Genexpressionslevels der Gene für *HMGA2* und *PPAR-gamma* in der Probe und
c) Eingruppieren des Spenders der Probe in eine von wenigstens vier Risikogruppen unter Berücksichtigung der Genexpressionslevels der Gene für *HMGA2* und *PPAR-gamma* in der Probe und des Körperfettanteils, insbesondere des BMI des Spenders, wobei die Einordnung in eine der Gruppen erfolgt ausgewählt aus der Gruppe bestehend aus
   a) erhöhtes relatives Genexpressionslevel für *HMGA2,* verringertes relatives Genexpressionslevel für *PPAR-gamma* und erhöhter Körperfettanteil insbesondere mittlerer BMI ≥ 25,
   b) verringertes relatives Genexpressionslevel für *HMGA2,* verringertes relatives Genexpressionslevel für *PPAR-gamma* und erhöhter Körperfettanteil insbesondere mittlerer BMI ≥ 25,
   c) verringertes relatives Genexpressionslevel für *HMGA2,* verringertes Genexpressionslevel für *PPAR-gamma* und nicht erhöhter Körperfettanteil insbesondere mittlerer BMI < 25,
   d) verringertes relatives Genexpressionslevel für *HMGA2,* erhöhtes relatives Genexpressionslevel für *PPAR-gamma* und nicht erhöhter Körperfettanteil insbesondere mittlerer BMI < 25,
wobei das Bereitstellen im Schritt a) so erfolgt, dass das Verfahren kein Diagnostizierverfahren ist, das am menschlichen oder tierischen Körper vorgenommen wird.

Bevorzugt umfasst das eingesetzte Fettgewebe weißes Fettgewebe, weiterbevorzugt besteht es daraus.

In dem erfindungsgemäßen Verfahren werden Individuen mit einem unreifen Fettgewebe identifiziert, basierend auf einer Genexpressionsanalyse, d.h. niedrige Level der *PPAR-*gamma-Expression sowie ein erhöhter Level der HMGA2-Expression weisen auf einen erhöhten

Anteil von unreifen Fettzellen im Fettgewebe hin. Umgekehrt deuten eine hohe *PPAR-*gamma-Expression sowie eine niedrige HMGA2-Expression auf einen erhöhten Anteil von reifen Fettzellen im Fettgewebe hin. Mittels der Kombination der Genexpression dieser beiden Gene ist es möglich, Aussagen über die Fettgewebszusammensetzung des Individuums zu treffen. Anhand dieser Wertekombination, können Rückschlüsse über u.a. das Risiko an Diabetes zu erkranken gezogen werden.

Überraschend hierbei ist, dass auf Basis des erfindungsgemäßen Verfahrens nicht nur zwei Risikogruppen, wie es aus der EP 2 682 752 A1 zu erwarten wäre, ableitbar sind, sondern wenigstens 4: Die genannte Offenlegungsschrift geht nämlich davon aus, dass sich die Expressionslevel von *HMGA2* und *PPAR*-gamma stets in einer signifikanten inversen Korrelation befinden. Dies würde bedeuten, dass aus Messung des Expressionslevels des einen Markers auch die möglichen Erkenntnisse gewonnen werden könnten, die aus Messung des Expressionslevels des anderen Markers gewonnen werden würden. Daher wäre der Fachmann davon abgehalten gewesen, schon aus Ökonomiegründen, für ein entsprechendes Verfahren beide Marker parallel zu messen. Überraschenderweise hat sich jedoch herausgestellt, dass sich die in der genannten Offenlegungsschrift behauptete Korrelation eben in Bezug auf das Risiko, an den genannten Erkrankungen zu erkranken, anders darstellt: Es gibt Fälle, die hinsichtlich der beiden Marker auf Expressionsebene eben nicht umgekehrt korreliert sind. Hierzu sei insbesondere auch auf die Beispiele verwiesen.

Der Begriff "Diagnose" bedeutet im Sinne dieses Textes eine Vorhersage einer erhöhten Wahrscheinlichkeit der Entwicklung und des Auftretens eines klinischen Zustandes oder einer Krankheit.

Der Begriff "Bestimmung" einer Krankheit bedeutet im Sinne dieses Textes, dass eine Krankheit, die bereits klinische Symptome zeigt, identifiziert wird.

"Diabetes" im Sinne des vorliegenden Textes umfasst insbesondere die Formen des Diabetes mellitus Typ I und Typ II bei Mensch und Tier, wobei besonders bevorzugt der Typ II beim Menschen gemeint ist.

"Herz-Kreislauf-Erkrankungen" im Sinne dieses vorliegenden Textes sind bevorzugt Koronare Herzkrankheit, Arteriosklerose, Hypertonie, Myokardinfarkt, periphere arterielle Verschlusskrankheit und Kardiomyopathie.

"Fettgewebe" im Sinne dieses Textes besteht aus in Bindegewebe eingelagerten Fettzellen (Adipozyten, Präadipozyten) sowie Immunzellen, Fibroblasten und Blutgefäßen.

Ein "Spender" im Sinne dieses Textes ist ein Mensch oder ein Tier, bevorzugt ein Mensch.

Das Bestimmen des Genexpressionslevels im Sinne der vorliegenden Erfindung kann auf jede dem Fachmann bekannte Weise erfolgen. Bevorzugt ist eine Bestimmung des Genexpressionslevels auf mRNA-Ebene oder auf Proteinebene.

"Risikogruppen" im Sinne dieses Textes sind solche Gruppen, die durch geeignete Unterscheidungsmerkmale voneinander getrennt werden können und jeweils ein gemeinsames erhöhtes oder nicht-erhöhtes Risiko in Bezug auf die Entwicklung oder das Vorhandensein einer Krankheit besitzen.

"HMGA2" im Sinne dieses Textes ist das High Mobility Group AT-Hook Protein 2 (HMGA2) bzw. dessen Gen bzw. die zugehörige mRNA und/oder Teile dieses Proteins bzw. Gens (bzw. dessen mRNA) bevorzugt wenigstens eine Aminosäurenkette ≥ 7 Aminosäuren, weiter bevorzugt ≥ 15 Aminosäuren und besonders bevorzugt ≥ 20 Aminosäuren bzw. eine Nucleinsäurenkette von ≥ 20 Nucleinsäuren, weiter bevorzugt ≥ 40 Nucleinsäuren und besonders bevorzugt ≥ 55 Nucleinsäuren ggf. pro Strang. HMGA2 ist ein Transkriptionsfaktor, der die Regulation der Genexpression beeinflusst und zur Gruppe der High Mobility Group A-Proteine (HMGA-Proteine) gehört. Die HMGA-Proteine sind Chromatin-assoziierte, säurelösliche Nicht-Histon-Proteine, die an sequenzunabhängige, spezifische Motive der DNA binden. Als architektonische Transkriptionsfaktoren erhöhen bzw. inhibieren sie über strukturelle Änderungen der Chromatinorganisation die Bindungsfähigkeit weiterer Transkriptionsfaktoren. Das humane HMGA2-Gen ist in der chromosomalen Region 12q14∼15 lokalisiert und besteht aus fünf Exons, die sich über einen ≥160kb langen Bereich erstrecken. Es codiert ein 109 Aminosäuren langes Protein, dessen molekulare Masse 12 kDa beträgt. Das HMGA2-Protein ist durch drei stark konservierte DNA-bindende Domänen, die sogenannten AT-Hooks und eine saure, negativ geladene C-terminale Domäne gekennzeichnet.

"PPAR-gamma" im Sinne dieses Textes ist der Peroxisom-Proliferator-aktivierte Rezeptor gamma (PPAR-gamma) bevorzugt PPAR-gamma Isoform 2 bzw. dessen Gen bzw. die zugehörige mRNA und/oder Teile dieses Proteins bzw. Gens (bzw. dessen mRNA) bevorzugt wenigstens eine Aminosäurenkette ≥ 7 Aminosäuren, weiter bevorzugt ≥ 15 Aminosäuren und besonders bevorzugt ≥ 20 Aminosäuren bzw. eine Nucleinsäurenkette von ≥ 20 Nucleinsäuren, weiter bevorzugt ≥ 40 Nucleinsäuren und besonders bevorzugt ≥ 55 Nucleinsäuren ggf. pro Strang. PPAR-gamma ist ein Liganden-bindender nukleärer Transkriptionsfaktor der PPAR-Unterfamilie, der zur Gruppe der nukleären Hormonrezeptoren gehört. Über Heterodimerisation mit dem Retinoid-X Rezeptor α (RXRα) aktiviert PPAR-gamma die Transkription verschiedener Gene. Das humane *PPAR-gamma-Gen* ist in der chromosomalen Bande 3p25 lokalisiert und besteht aus 11 Exons. Das humane *PPAR-gamma-Gen* codiert für 3 Isoformen, die jeweils ein 477, ein 505 und ein 186 Aminosäuren langes Protein darstellen.

Im Sinne dieses Textes wird der Körperfettanteil bevorzugt mittels nachfolgender Methode bestimmt: BMI (Body-Mass-Index), ABSI (A Body Shape Index), WHR (Waist-To-Hip-Ratio), WtHR (Waist-to-Height-Ratio), Körperfettmasse, subkutane Fettgewebsmasse, viszerale Fettgewebsmasse, UWW (Under Water Weighing), ADP (Air Displacement Plethysmography), Calipometrie, DEXA (Dual Energy X-ray Absorptiometry), BIA (Bioelektrische Impendanzanalyse), BIVA (Bioelectrical Impedance Vector Analysis) und MRI (Magnet Resonance Imaging), wobei der BMI bevorzugt ist.

Bevorzugt im Sinne der vorliegenden Erfindung ist, dass der Spender ein Mensch und/oder die Krankheit Diabetes mellitus Typ II ist.

Das den Erfindern vorliegende Zahlenmaterial und die experimentelle Erfahrung haben gezeigt, dass sich das erfindungsgemäße Verfahren insbesondere für die Prognose und/oder Diagnose der Krankheit Diabetes mellitus Typ II eignet.

Bevorzugt ist für das erfindungsgemäße Verfahren, dass die Probe durch Punktion des subkutanen abdominalen Fettgewebes gewonnen wurde. Durch fächerförmiges Punktieren unter Sog lassen sich besonders gut Zellen und Zellverbände gewinnen, die eine molekulargenetische Analyse ermöglichen. Das fächerförmige Vorgehen bei der Punktion vermindert zum einen eine Verklebung/Verstopfung der Kanülenspitze mit Fettzellen zum anderen erhält man Zellen aus verschiedenen Regionen des betreffenden Fettgewebes und hat somit einen repräsentativen Querschnitt der Verteilung unterschiedlicher Fettgewebszelltypen.

Besonders bevorzugt ist ein erfindungsgemäßes Verfahren, wobei die Probemasse ≤ **50** mg, bevorzugt ≤ **20** mg und besonders bevorzugt ≤ 5 mg ist.

Überraschenderweise hat sich gezeigt, dass selbst bei sehr kleinen Probenvolumen zuverlässig differenzierte Ergebnisse erzielt werden können. Dabei ist es besonders bevorzugt, dass die Probe durch Punktion als Feinnadel-Aspirat gewonnen wurde, wie es im Beispiel 1 unter Feinnadel-Aspiration beschrieben ist.

Erfindungsgemäß bevorzugt wird das Verfahren so durchgeführt, dass die Bestimmung des Genexpressions-Levels auf mRNA-Ebene erfolgt.

Für die Bestimmung auf mRNA-Ebene gibt es eine Vielzahl von Techniken. Trotzdem ist es überraschend, dass auf mRNA-Ebene auch bei sehr geringen Probenmengen zuverlässige und differenzierte Ergebnisse mit der erfindungsgemäßen Methode erzielt werden.

Für das Bestimmen der Genexpressions-Level auf mRNA-Ebene sind üblicherweise die Schritte RNA-Isolierung oder wenigstens Anreicherung, nachfolgende cDNA-Synthese und darauffolgend eine quantitative PCR üblich.

Für die RNA-Isolierung können alle dem Fachmann geläufige Verfahren eingesetzt werden, ebenso für die cDNA-Synthese.

Bevorzugte Verfahren für die Quantifizierung der Genexpression sind Array-Hybridisierung (DNA- bzw. Protein-Arrays), Massenspektrometrie (MALDI-TOF), serielle Analyse der Genexpression (SAGE) und andere Methoden zur Detektion von mRNAs oder Proteinen oder Fragmenten davon, insbesondere bevorzugt ist dabei eine quantitative Real-Time PCR (qRT-PCR).

Grundsätzlich sind jedoch alle Verfahren möglich, die zu einer zuverlässigen Genexpressionsmessung führen.

Bevorzugt ist ein erfindungsgemäßes Verfahren, wobei vor dem Ausführen der Real-Time PCR eine Prä-Amplifizierung der cDNA erfolgt.

Hierdurch lassen sich noch genauere Ergebnisse gewinnen, die zudem auch reproduzierbar sind.

Besonders bevorzugt ist für das erfindungsgemäße Verfahren, dass die Eingruppierung in Schritt c) unter Verwendung des multivariaten Modells der selbstorganisierenden Karten nach Kohonen erfolgt.

Zur Anwendung des Multivariatenmodells der selbstorganisierten Karten nach Kohonen wird auch auf Beispiel 3 verwiesen. Überraschenderweise hat sich herausgestellt, dass sich mit der Einordnung mittels der genannten statistischen Methode sehr deutlich vier Spendergruppen heraus entwickeln. Dies war nach dem Stand der Technik nicht zu erwarten gewesen. Hierzu wird auf die oben gemachten Ausführungen verwiesen.

Bevorzugt ist ein erfindungsgemäßes Verfahren, wobei die Einordnung in eine der Gruppen erfolgt ausgewählt aus der Gruppe bestehend aus
a) erhöhtes relatives Genexpressionslevel für *HMGA2,* verringertes relatives Genexpressionslevel für *PPAR-gamma* und erhöhter Körperfettanteil insbesondere mittlerer BMI ≥ 25, bevorzugt ≥ 28, weiter bevorzugt ≥ 30,
b) verringertes relatives Genexpressionslevel für *HMGA2,* verringertes relatives Genexpressionslevel für *PPAR-gamma* und erhöhter Körperfettanteil insbesondere mittlerer BMI ≥ 25, bevorzugt ≥ 28, weiter bevorzugt ≥ 30,
c) verringertes relatives Genexpressionslevel für *HMGA2,* verringertes Genexpressionslevel für *PPAR-gamma* und nicht erhöhter Körperfettanteil insbesondere mittlerer BMI < 25 und bevorzugt < 23,
d) verringertes relatives Genexpressionslevel für *HMGA2,* erhöhtes relatives Genexpressionslevel für *PPAR-gamma* und nicht erhöhter Körperfettanteil insbesondere mittlerer BMI < 25 bevorzugt < 23,
e) erhöhtes relatives Genexpressionslevel für *HMGA2,* verringertes Genexpressionslevel für *PPAR-gamma* und nicht erhöhter Körperfettanteil insbesondere mittlerer BMI < 25 und bevorzugt < 23,
f) verringertes relatives Genexpressionslevel für *HMGA2,* erhöhtes relatives Genexpressionslevel für *PPAR-gamma* und erhöhter Körperfettanteil insbesondere mittlerer BMI ≥ 25, bevorzugt ≥ 28, weiter bevorzugt ≥ 30.

Diese sechs Gruppen lassen gegenüber dem bislang im Stand der Technik möglichen Aussagen zusätzlich den Informationsgewinn zu:
dabei ist die Gruppe a) einem erhöhten Risiko ausgesetzt u.a. an Hyperinsulinämie, Hyperglykämie, Insulinresistenz, Diabetes mellitus Typ II zu erkranken bzw. schon unerkannt daran erkrankt zu sein.

dabei ist die Gruppe b) einem normalen bzw. leicht erhöhten Risiko ausgesetzt u.a. an Hyperinsulinämie, Hyperglykämie, Insulinresistenz, Diabetes mellitus Typ II zu erkranken bzw. schon unerkannt daran erkrankt zu sein.

dabei ist die Gruppe c) einem erhöhten Risiko ausgesetzt u.a. an Insulinresistenz, Diabetes mellitus Typ IIAdipositas, zu erkranken bzw. schon unerkannt daran erkrankt zu sein.

dabei ist die Gruppe d) einem niedrigen Risiko ausgesetzt u.a. Insulinresistenz, Diabetes mellitus Typ II, Adipositas zu erkranken bzw. schon unerkannt daran erkrankt zu sein.

dabei ist die Gruppe e) einem erhöhten Risiko ausgesetzt u.a. an Hyperinsulinämie, Hyperglykämie, Insulinresistenz, Diabetes mellitus Typ II, zu erkranken bzw. schon unerkannt daran erkrankt zu sein.

dabei ist die Gruppe f) einem niedrigen Risiko ausgesetzt u.a. an Hyperinsulinämie, Hyperglykämie, Insulinresistenz, Diabetes mellitus Typ II, Adipositas, zu erkranken bzw. schon unerkannt daran erkrankt zu sein.

Insbesondere die Gruppen c) und e) stellen dabei zwei im Stand der Technik nicht vorgeschriebene Risikogruppen dar.

"Erhöhtes relatives Genexpressions-Level" bedeutet in diesem Zusammenhang, dass das Genexpressions-Level gegenüber dem Mittelwert der Genexpressions-Levels eines Patientenkollektivs (bevorzugt n größer 100) erhöht ist. Im Rahmen der relativen Quantifizierung wird die Expression der Zielgene in Relation zu einer sogenannten endogenen Kontrolle ermittelt. Als endogene Kontrolle dienen ubiquitär exprimierte Housekeeping-Gene, bevorzugt ausgewählt aus der Gruppe bestehend aus *HPRT, 18S, GAPDH, GUSB, PBGD, B2M, ABL, RPLP0,* wobei ganz besonders *HPRT* bevorzugt ist.

Bevorzugt gilt ein solcher Genexpressions-Levelwert als erhöht, wenn er um wenigstens 5%, weiter bevorzugt wenigstens 10%, besonders bevorzugt wenigstens 20% vom jeweiligen Bezugswert nach oben abweicht.

Ein nicht-erhöhtes Genexpressions-Level im Sinne der vorliegenden Anmeldung ist demgegenüber ein solches, das der gegenüber einem oder mehreren der Bezugswerte verringert ist, das bedeutet, um wenigstens 5%, bevorzugt wenigstens 10% und weitere bevorzugt wenigstens 20% verringert ist. Teil der vorliegenden Offenbarung, aber nicht Teil der Erfindung, wie in den Ansprüchen definiert, ist auch ein Kit für ein erfindungsgemäßes Verfahren, umfassend
a) ein Primerpaar, das an die cDNA (Primer kann auch an DNA binden) von *HMGA2* bindet und
b) ein Primerpaar, das an die cDNA (Primer kann auch an DNA binden) von *PPAR-gamma* bindet.

Ein Primer, der an eine bestimmte cDNA bindet, ist dabei ein solcher, der aufgrund seiner Nukleotidsequenz unter stringenten Bedingungen für eine Hybridisation an die Ziel-cDNA binden kann.

Stringente Bedingungen sind hierbei definiert als äquivalent zur Hybridisierung in 6x SSC bei 45 °C gefolgt von einem Waschschritt in 0,2x SSC, 0,1 % SDS bei 65 °C (Current Protocols in Molecular Biology, John Wiley & Sons, N.Y., 6.3.1-6.3.6, 1991).

Ein Primerpaar sind hierbei 2 Primer, die an Abschnitte der jeweiligen cDNA an verschiedene Stränge unter stringenten Bedingungen binden.

Bevorzugt kann das erfindungsgemäße Kit einen oder mehrere weitere der nachfolgend aufgezählten Komponenten enthalten:
Fluoreszenz-markierte DNA-Sonden, Reverse Transkriptasen, Polymerasen, Hexanukleotide, Nukleotide, RNase-lnhibitoren, Spritzen und Kanülen.

In den folgenden Beispielen wird Übergewicht als ein BMI >25 definiert.

Fettgewebsproben wurden entweder mittels Feinnadel-Aspiration oderwährend einer Operation gewonnen. Nach der RNA-Isolierung, cDNA-Synthese und cDNA-Präamplifikation wurde die Expression der mRNA von *HMGA2* und PPAR-gamma mittels quantitativer Real-Time-PCR bestimmt.

Sofern in den Beispielen vom Mittelwert gesprochen wird ist im Zweifelsfall der arithmetische Mittelwert gemeint.

### Beispiel 1

### Nachweis der Expression von PPAR-gamma und HMGA2 in Fettgewebspunktaten, gewonnen durch Feinnadel-Aspiration des subkutanen abdominalen Fettgewebes

### Material und Methoden

### Feinnadel-Aspiration

Feinnadel-Aspirate wurden durch Punktion von subkutanem abdominalem hWAT mittels 20 ml Spritze und einer Einmal-Injektions-Kanüle (Durchmesser 0,90 x 40 mm) gewonnen. Nach Desinfektion der Einstichstelle wurde die Kanüle in das Unterhaut-Fettgewebe eingeführt. Mit der Spritze wurde ein Unterdruck erzeugt und die Kanüle im Gewebe fächerförmig vor und zurück bewegt, um so Zellen des Fettgewebes zu aspirieren. Direkt nach der Punktion wurden die Proben in 1 ml QIAzol Lysis Reagenz (QIAGEN, Hilden, Deutschland) aufgenommen und die Kanüle mehrmals mit dem QIAzol gespült. Anschließend wurden die Proben bei -80 °C eingefroren.

### RNA-Isolierung

Die Isolierung der Gesamt-RNA erfolgte mittels RNeasy Lipid Tissue Mini Kit (QIAGEN, Hilden, Deutschland) in einem QIAcube (QIAGEN, Hilden, Deutschland) nach Herstellerangaben. Die Feinnadel-Aspirate (5mg) in 1 ml QIAzol Lysis Reagenz wurden in einem Tissue Lyser II (QIAGEN, Hilden, Deutschland) homogenisiert und anschließend wurde das Homogenat bei Raumtemperatur für 5 min inkubiert. Es folgte die Zugabe von 200 µl Chloroform, welches mittels kräftigem Schütteln per Hand für 15 sec mit der Probe vermischt wurde. Die Probe wurde erneut für 2 min bei Raumtemperatur inkubiert und mit 12.000 x g zentrifugiert für 15 min bei 4 °C. Anschließend wurde die obere wässrige Phase in ein neues 2 ml Cup transferiert und die Gesamt-RNA wurde über eine Qiagen RNeasy Mini Spin Säule (QIAGEN, Hilden, Deutschland) in einem QIAcube nach Herstellerangaben isoliert.

### cDNA-Synthese

Für die cDNA-Synthese wurde ≤ 250 ng RNA mittels 200 U M-MLV Reverse Transkriptase, RNase Out (Invitrogen, Darmstadt, Germany) und 150 ng Random-Primer (Invitrogen, Darmstadt, Germany) nach Herstellerangaben in cDNA umgeschrieben. Die RNA wurde bei 65 °C für 5 min denaturiert und anschließend mindestens 1 min auf Eis gelagert. Nach der Zugabe des Enzyms wurde für das Annealing der Random-Primer an die RNA der Mix für 10 min bei 25 °C inkubiert. Die anschließende Reverse Transkription wurde bei 37 °C für 50 min durchgeführt, gefolgt von einer 15 min Inaktivierung der Reversen Transkriptase bei 70 C.

### Prä-Amplifizierung der cDNA

5 µl cDNA wurde mittels RealTime ready cDNA Preamp Mastermix (Roche, Mannheim, Germany) unter Verwendung von *HMGA2* und *HPRT* (Hypoxanthin-Phosphoribosyltransferase 1) spezifischen Primern nach Herstellerangaben präamplifiziert. Die Prä-Amplifikation der cDNA erfolgte nach folgendem Temperaturprofil: 95 °C für 1 min gefolgt von 14 Zyklen bei 95 °C für 15 sec und bei 60 °C für 4 min.

### Quantitative Real-Time PCR (qRT-PCR)

Die relative Quantifizierung der Genexpression wurde mittels Real-Time PCR auf dem Applied Biosystems 7300 Real-Time PCR System durchgeführt. Kommerziell erhältliche Genexpressions-Assays (Life Technologies, Carlsbad, CA, USA) wurden zur Quantifizierung der mRNA-Level von *HMGA2* (Assay-ID Hs00171569_m1) und *PPAR-gamma* (Assay-ID Hs01115513_m1) verwendet. Als endogene Kontrolle wurde, wie von Klemke et al. (Klemke M, Meyer A, Hashemi Nezhad M, Beige G, Bartnitzke S, Bullerdiek J (2010) Loss of let-7 binding sites resulting from truncations of the 3' untranslated region of HMGA2 mRNA in uterine leiomyomas. Cancer Genet Cytogenet 196:119-123) beschrieben, *HPRT* genutzt. Alle gemessenen Proben wurden dreifach bestimmt. Die Quantifizierung der Genexpression wurde in 96-Well Platten mit der zu untersuchenden präamplifizierten cDNA, den jeweiligen genspezifischen Assays und dem FastStart Universal Probe Master (Rox) (Roche, Mannheim, Germany) durchgeführt. Das Temperaturprofil der Real-Time PCR folgte den Herstellerangaben: Bei 95 °C erfolgt eine Denaturierung des Templates für 10 min. Anschließend folgte die Amplifikation in 50 Zyklen beginnend mit der Denaturierung für 15 sec bei 95 °C und der Kombination aus Annealing/Elongation für 60 sec bei 60 °C. Die erhaltenen Daten wurden mittels komparativer Delta-Ct-Methode (ΔΔCT-Methode) ausgewertet (Livak KJ, Schmittgen TD (2001) Analysis of relative gene expression data using real-time quantitative PCR and the 2 -ΔΔC(T) Method. Methods 25: 402-408).

### Ergebnisse

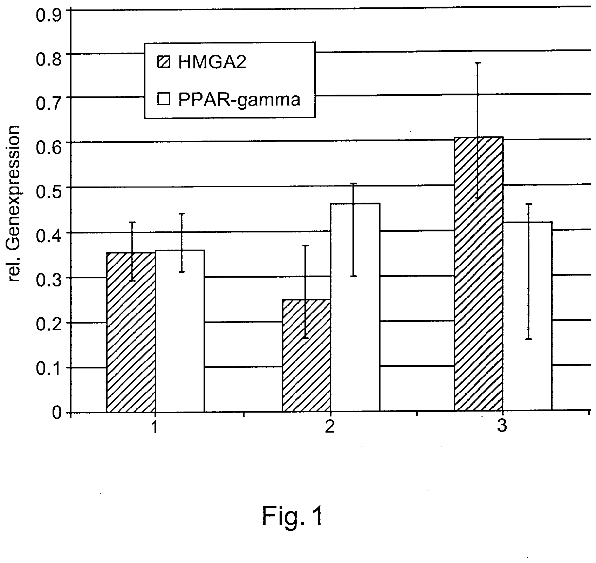

Die Genexpression von *PPAR-gamma* und *HMGA2* konnte mittels qRT-PCR in drei Proben gemessen werden (Fig. 1). Die Ergebnisse zeigen eindeutig, dass HMGA2-mRNA zuverlässig aus Feinnadel-Aspiraten mit selbst sehr geringen Mengen an Fettgewebszellen quantifizierbar ist. Zudem reicht die Menge der isolierten RNA aus den Feinnadel-Aspiraten aus, um die Expression von weiteren Genen nach einer Prä-Amplifikation zu bestimmen.

Fig.1 stellt die relative Expression von *HMGA2-* und *PPAR*-gamma in drei Proben dar. Sie zeigt, dass eine zuverlässige Quantifizierung der *HMGA2-* und PPAR-gamma-Expression selbst aus sehr geringen Mengen von RNA (Konzentration ≤ 25 ng/µl), die durch Feinnadel-Aspiration gewonnen wurde, gelingt.

### Beispiel 2

### Die Expression von HMGA2 und PPAR-gamma in Fettgewebsproben von übergewichtigen Individuen

### Material und Methoden

### Gewebeproben

Die humanen subkutanen abdominalen Fettgewebe wurden während Operationen entnommen und nach der Operation in flüssigem Stickstoff gelagert. Für alle verwendeten humanen Fettgewebsproben wurden die Forderungen der Deklaration von Helsinki erfüllt. Eine schriftliche Einverständniserklärung für die Verwendung der Gewebeproben wurde von den Patienten (n = 157) abgegeben.

### RNA-Isolierung

Die Isolierung der Gesamt-RNA erfolgte mittels RNeasy Lipid Tissue Mini Kit (QIAGEN, Hilden, Deutschland) wie unter Beispiel 1 beschrieben.

### cDNA-Synthese

Die cDNA-Synthese erfolgte ebenfalls wie unter Beispiel 1 beschrieben.

### Quantitative Real-Time PCR

Auch die Durchführung der qRT-PCR erfolgt wie in Beispiel 1 beschrieben.

### Statistische Analyse

Die statistische Signifikanz der Unterschiede zwischen der normalgewichtigen und der übergewichtigen Gruppe wurde mittels Kendalls-Tau-b-Test bestimmt. In allen Vergleichen wurde p < 0,05 als statistisch signifikant und p < 0,001 als höchst signifikant angesehen.

### Ergebnisse

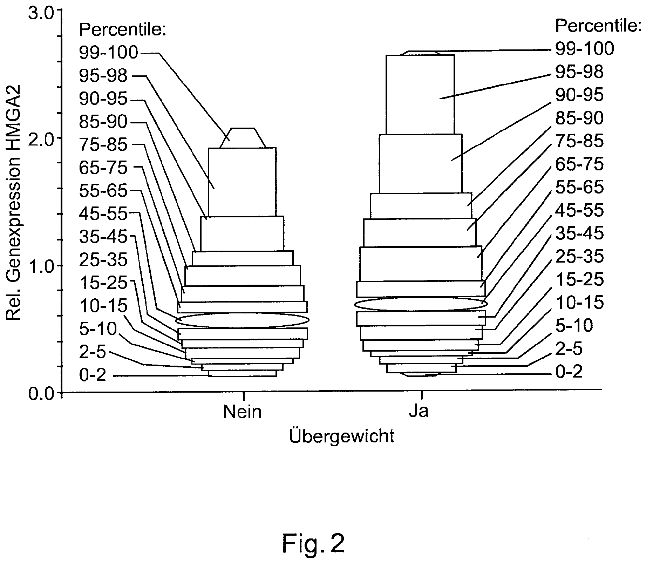

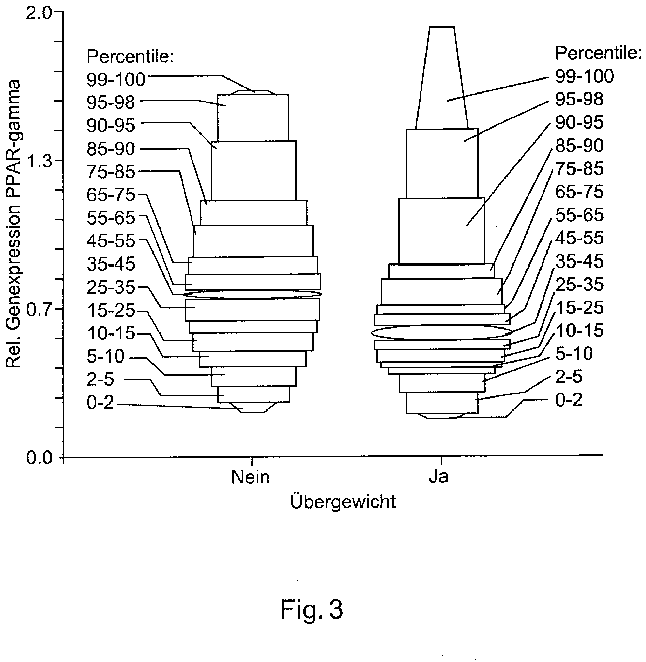

Statistisch signifikante Unterschiede (p < 0,05) in der Expression von *HMGA2* konnten in Proben von normalgewichtigen Individuen (n = 74) im Vergleich zu übergewichtigen Individuen (n = 83) erkannt werden (Fig. 2). Des Weiteren wurden höchst signifikante Unterschiede der PPAR-gamma-mRNA Level in Proben von normalgewichtigen Individuen (n = 74) im Vergleich zu übergewichtigen Individuen (n = 83) festgestellt (Fig. 3).

Fig. 2 zeigt die HMGA2-Expression normal- und übergewichtiger Individuen als Perzentilen-Plot. Die 15 Perzentilen sind folgendermaßen voneinander abgegrenzt: 0-2, 2-5, 5-10, 10-15, 15-25, 25-35, 35-45, 45-55, 55-65, 65-75, 75-85, 85-90, 90-95, 95-99, 99-100.

Aus Fig. 2 geht hervor, dass fast jede Perzentile in der übergewichtigen Gruppe eine höhere Genexpression aufweist als die korrespondierende Perzentile der jeweiligen normalgewichtigen Gruppe.

Fig. 3 zeigt die PPAR-gamma-Expression normal- und übergewichtiger Individuen als Perzentilen-Plot. Die 15 Perzentilen sind folgendermaßen voneinander abgegrenzt: 0-2, 2-5, 5-10, 10-15, 15-25, 25-35, 35-45, 45-55, 55-65, 65-75, 75-85, 85-90, 90-95, 95-99, 99-100.

Fig. 3 zeigt, dass fast jede Perzentile in der normalgewichtigen Gruppe eine höhere Genexpression aufweist als die korrespondierende Perzentile der jeweiligen übergewichtigen Gruppe.

### Beispiel 3

### Datenanalyse mittels Selbstorganisierender Karten zeigt vier unterschiedliche PPAR-gamma- und HMGA2-Genexpressionsprofile

### Material und Methoden

### Gewebeproben

Die humanen subkutanen abdominalen Fettgewebe wurden während Operationen entnommen und nach der Operation in flüssigem Stickstoff gelagert. Für alle verwendeten humanen Fettgewebsproben wurden die Forderungen der Deklaration von Helsinki erfüllt. Eine schriftliche Einverständniserklärung für die Verwendung der Gewebeproben wurde von den Patienten (n = 157) gegeben.

### Weitere Schritte

Die RNA-Isolierung, die cDNA-Synthese und die qRT-PCR erfolgten wie in Beispiel 2 beschrieben.

### Statistische Analyse

Um die Beziehung der Biomarker *HMGA2* und *PPAR-gamma* mit dem BMI zu analysieren, wurde Kohonens multivariates Modell der Selbstorganisierenden Karten (vergl. Kohonen T (2001) Self-Organizing Maps. Third, extended edition. Springer Berlin, Heidelberg, New York) verwendet. Das Ziel dieser Analyse war es, die Probanden/Patienten so in Gruppen (Segmente) einzuteilen, dass die Varianz der Werte von BMI, *HMGA2-* und *PPAR-gamma-*Expression innerhalb der Segmente minimal und zwischen den Segmenten maximal ist.

### Ergebnisse

Nach der Analyse der Beziehung der Biomarker *HMGA2, PPAR-gamma* und dem BMI-Status mittels Kohonens selbstorganisierenden Karten (SOM) zeigte sich überraschenderweise eine Einteilung des Patientenkollektivs in vier Gruppen und nicht wie erwartet in zwei Gruppen, also in eine normalgewichtige und eine übergewichtige Gruppe.

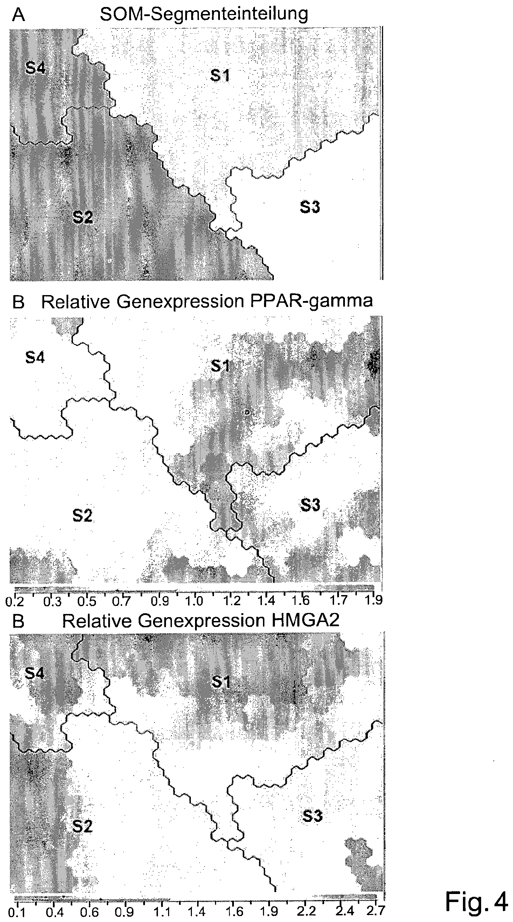

Die Fig. 4A zeigt die Einteilung des Patientenkollektives mittels SOM nach Kohonen unter Berücksichtigung der relativen *HMGA2-* und PPAR-gamma-Expression sowie BMI-Status. Zwei Gruppen (S1 und S3) haben ein BMI-Mittelwert > 30 und weisen ausgeprägte Unterschiede in den Expressionsprofilen von *HMGA2* und *PPAR-gamma* auf. Die Patienten mit einem BMI-Mittelwert von < 25 wurden ebenso in zwei Gruppen eingeteilt und zeigten ebenfalls jeweils ein eigenes Genexpressionsprofil von *HMGA2* und *PPAR-gamma.* Somit konnte gezeigt werden, dass die in der EP 2 682 752 A1 dargestellte inverse Korrelation von *HMGA2* und *PPAR-gamma* nur teilweise zutrifft. Insbesondere ist mit den hier beschriebenen Methoden eine sehr viel präzisere Einteilung aufgrund der Genexpressionsprofile der Patienten in potentielle Risikogruppen möglich. In den Segmenten S1 und S3 befinden sich hauptsächlich Patienten mit Übergewicht, wohingegen in den Segmenten S2 und S4 sich Patienten mit Normalgewicht befinden.

Die Fig. 4B zeigt die Einteilung des Patientenkollektives mittels SOM nach Kohonen unter Berücksichtigung des BMI-Status in Bezug auf die relative PPAR-gamma-Expression (geringe Expression=hell; hohe Expression= dunkel).

Die Fig. 4C zeigt die Einteilung des Patientenkollektives mittels SOM nach Kohonen unter Berücksichtigung des BMI-Status in Bezug auf die relative *HMGA2*-Expression (geringe Expression=hell; hohe Expression= dunkel).

Die Selbstorganisierende Karte für *PPAR-gamma* (Fig. 4B) zeigt eine erhöhte *PPAR-gamma*-Expression bei normalgewichtigen Patienten im Segment S4. Im Gegensatz dazu zeigen übergewichtige Patienten im Segment S3 eine hohe *HMGA2*-Expression (Fig. 4C).

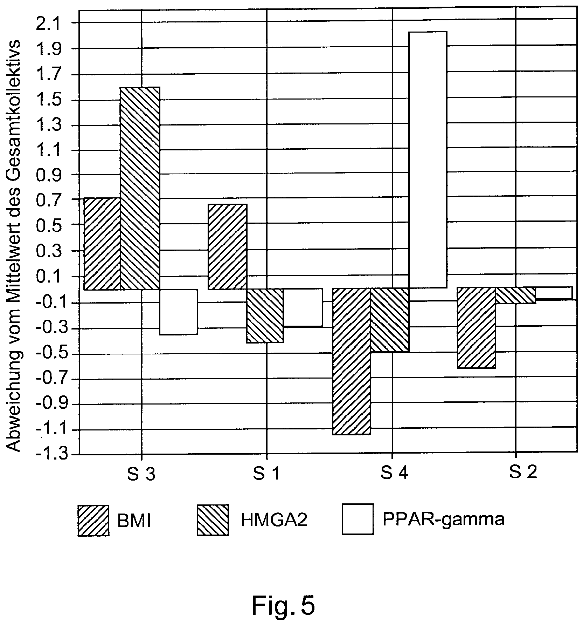

Fig. 5: zeigt ein Balkendiagramm der Attribute BMI, *HMGA2-* und *PPAR-gamma-*Expression in den vier Segmenten. Die Höhe eines Balkens in dem Balkendiagramm ist die Abweichung des Mittelwerts des Attributwerts innerhalb des Segments von dem jeweiligen Mittelwert des gesamten Datensatzes. Die Einheit entspricht der Standardabweichung vom gesamten Datensatz.

In Fig. 5 sind die Abweichungen der Mittelwerte der jeweiligen Attribute eines Segments von den Mittelwerten des jeweiligen Attributs des gesamten Datensatzes dargestellt.

In Fig. 5 ist Segment S4 charakterisiert durch eine erhöhte Expression von *PPAR-gamma,* wohingegen die Segmente S1, S2 und S3 eine geringe Expression von *PPAR-gamma* aufweisen. Das Segment S3 zeigt eine hohe *HMGA2*-Expression im Gegensatz zu den Segmenten S1, S2 und S4, die eine niedrige *HMGA2*-Expression aufweisen.

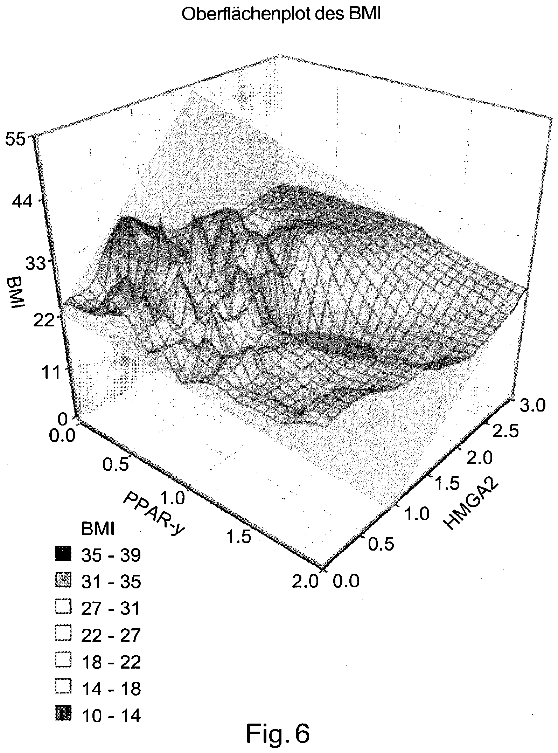

Fig. 6: Surface-Plot erstellt aus den Daten für BMI, *HMGA2-* und PPAR-gamma-Expression in humanem subkutanem abdominalem weißem Fettgewebe (n =157). Niedrige *PPAR-gamma-* (≤ 0,671) und hohe *HMGA2*-Expression (≥ 0,717) korrelieren mit einem hohen BMI und umgekehrt.

### Beispiel 4

### Einfluss von Gewichtsveränderungen auf die Genexpressionsprofile von HMGA2 und PPAR-gamma

### Material und Methoden

### Probenvorbereitung

Die Probengewinnung erfolgte mittels Feinnadel-Aspiration wie in Beispiel 1 beschrieben. Auch die nachfolgenden Schritte der Probenaufbereitung, also RNA-Isolierung, cDNA-Synthese, Prä-Amplifizierung der cDNA und die quantitative Real-Time-PCR erfolgten wie in Beispiel 1 beschrieben.

### Ergebnisse

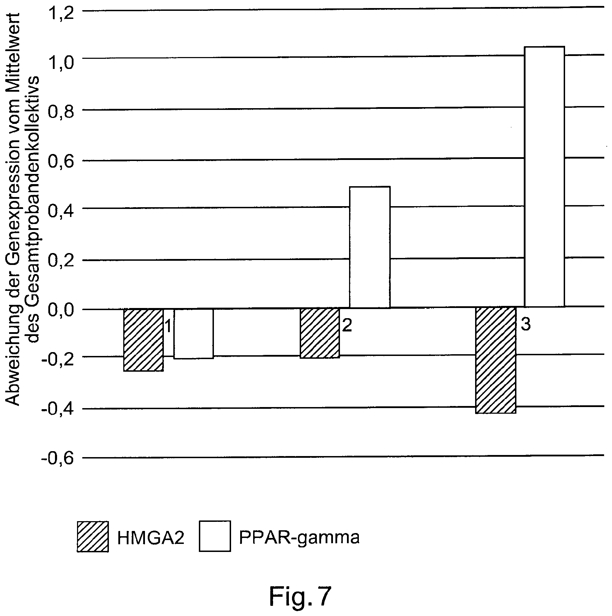

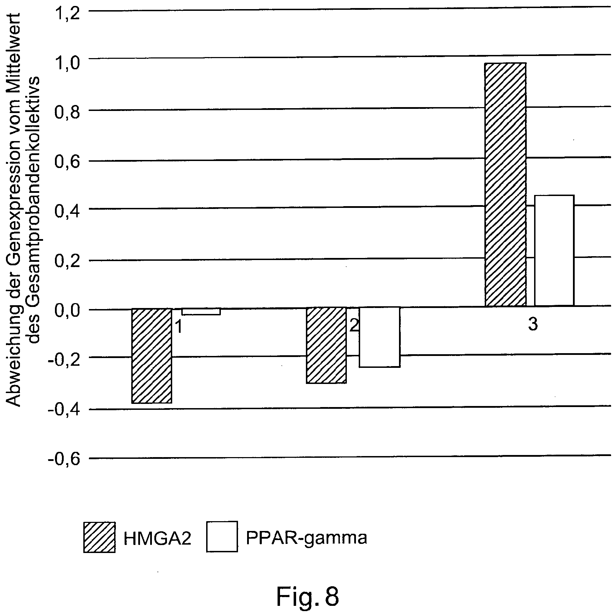

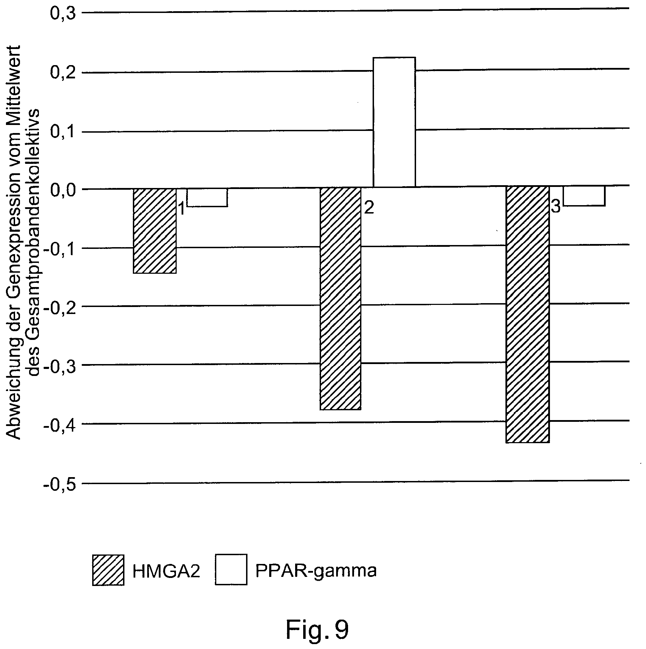

Eine Abnahme des Körpergewichts und des BMIs der Probanden 1 und 2 über einen Zeitraum von 3 Monaten führt zu Veränderungen in dem Genexpressionsprofil von *HMGA2* und *PPAR-gamma* (Figuren 7 u. 8). Proband 1 (Fig. 7) reduzierte vom 1. bis zum 3. Messzeitpunkt sein Körpergewicht um - 2,7 kg und seinen BMI von 23,4 auf 22,5, gleichzeitig änderte sich seine Abweichung der Genexpression vom Mittelwert des Gesamt-Probandenkollektivs von *HMGA2* und *PPAR-gamma* von -0,253 und -0,207 (1. Messzeitpunkt) auf -0,427 bzw. 1,039 (3. Messzeitpunkt). Vom 2. Messpunkt zum 3. Messpunkt veränderte sich der Körperfettanteil zudem um - 0,9%. Proband 2 (Fig. 8) reduzierte vom 1. bis zum 3. Messzeitpunkt sein Körpergewicht um - 1,8 kg und seinen BMI von 31,2 auf 30,5, gleichzeitig änderte sich seine Abweichung der Genexpression vom Mittelwert des Gesamt-Probandenkollektivs von *HMGA2* und *PPAR-gamma* von - 0,381 und -0,033 (1. Messzeitpunkt) auf 0,971 bzw. 0,440 (3. Messzeitpunkt). Vom 2. Messpunkt zum 3. Messpunkt veränderte sich der Körperfettanteil zudem um + 1,8%. Bei Proband 3 (Fig. 9) veränderte sich vom 1. bis zum 3. Messzeitpunkt das Körpergewicht um + 0,2 kg und sein BMI von 41,5 auf 41,6, gleichzeitig änderte sich seine Abweichung der Genexpression vom Mittelwert des Gesamt-Probandenkollektivs von *HMGA2* und *PPAR-gamma* von -0,145 und - 0,034 (1. Messzeitpunkt) auf -0,437 bzw. -0,036 (3. Messzeitpunkt). Vom 2. Messpunkt zum 3. Messpunkt veränderte sich der Körperfettanteil zudem um - 1,3%.

Diese Ergebnisse zeigen, dass die Fettgewebszusammensetzung von reifen und unreifen Fettzellen durch Veränderung des Körpergewichtes beeinflussbar ist. Diese Effekte lassen sich aber nicht einfach durch Messungen des Körpergewichts, BMI oder des Körperfettanteils bestimmen, sondern es bedarf u.a. einer Analyse der Genexpressionsprofile um eine Änderung der Fettgewebszusammensetzung zu bestimmen.

Fig. 7 ist die Darstellung der Abweichung der Genexpression von *HMGA2* und *PPAR-gamma* des Probanden 1 vom jeweiligen Mittelwert der Genexpression von *HMGA2* und *PPAR-gamma* des gesamten Probandenkollektivs (n = 157) an den drei Messzeitpunkten (Gesamtmesszeitraum 3 Monate, Abstand zwischen den einzelnen Messzeitpunkten jeweils ein Monat). Die Abweichung der Genexpression von *HMGA2* betrug zu den folgenden Messzeitpunkten: 1. -0,253; 2. -0,207; 3. -0,427. Die Abweichung der Genexpression von *PPAR-gamma* betrug zu den folgenden Messzeitpunkten: 1. -0,207; 2. 0,492; 3. 1,039.

Fig. 8 ist die Darstellung der Abweichung der Genexpression von *HMGA2* und *PPAR-gamma* des Probanden 2 vom jeweiligen Mittelwert der Genexpression von *HMGA2* und *PPAR-gamma* des gesamten Probandenkollektivs (n = 157) an den drei Messzeitpunkten (Gesamtmesszeitraum 3 Monate, Abstand zwischen den einzelnen Messzeitpunkten ungefähr jeweils ein Monat). Die Abweichung der Genexpression von *HMGA2* betrug zu den folgenden Messzeitpunkten: 1. -0,381; 2. -0,297; 3. 0,971. Die Abweichung der Genexpression von *PPAR-gamma* betrug zu den folgenden Messzeitpunkten: 1. -0,033; 2. -0,245; 3. 0,440.

Fig. 9 ist die Darstellung der Abweichung der Genexpression von *HMGA2* und *PPAR-gamma* des Probanden 3 vom jeweiligen Mittelwert der Genexpression von *HMGA2* und *PPAR-gamma* des gesamten Probandenkollektivs (n = 157) an den drei Messzeitpunkten (Gesamtmesszeitraum 3 Monate, Abstand zwischen den einzelnen Messzeitpunkten ungefähr jeweils ein Monat). Die Abweichung der Genexpression von *HMGA2* betrug zu den folgenden Messzeitpunkten: 1. -0,145; 2. -0,381; 3. -0,437. Die Abweichung der Genexpression von *PPAR-gamma* betrug zu den folgenden Messzeitpunkten: 1. -0,034; 2. 0,221; 3. -0,036.

### Beispiel 5

### Expressionsprofile von PPAR-gamma und HMGA2 im Fettgewebe von Diabetikern

### Material und Methoden

### Gewebeproben

Die humanen subkutanen abdominalen Fettgewebe wurden während Operationen entnommen und nach der Operation in flüssigem Stickstoff gelagert. Für alle verwendeten humanen Fettgewebsproben wurden die Forderungen der Deklaration von Helsinki erfüllt. Eine schriftliche Einverständniserklärung für die Verwendung der Gewebeproben wurde von den Patienten gegeben.

Die RNA-Isolierung, die cDNA-Synthese und die quantitative Real-Time-PCR wurden durchgeführt, wie unter Beispiel 1 beschrieben.

### Ergebnisse

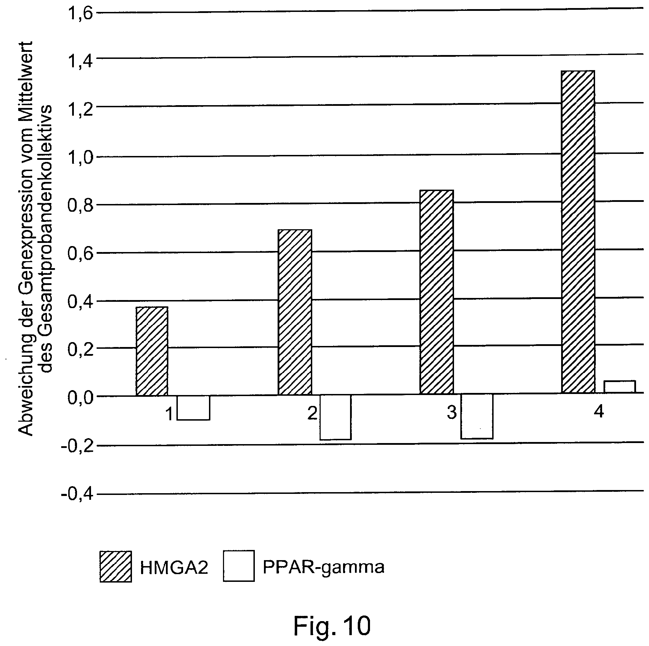

Fig. 10 zeigt ein Säulendiagramm der Genexpressionsprofile von *PPAR-gamma und HMGA2,* erstellt aus der Abweichung der Mittelwerte der Expression des entsprechenden Gens vom jeweiligen Mittelwert der Genexpression des Gesamtprobandenkollektivs von 4 Diabetikern mit Diabetes mellitus Typ II. Die Abweichung der Genexpression von *HMGA2* des jeweiligen Diabetikers vom Mittelwert des Gesamtprobandenkollektivs betrug: 1: 0,375; 2: 0,692; 3: 0,847; 4: 1,344. Die Abweichung der Genexpression von *PPAR-gamma* des jeweiligen Diabetikers vom Mittelwert des Gesamtprobandenkollektivs betrug: 1: -0,105; 2: -0,197; 3: -0,185; 4: 0,049.

Die Genexpressionsprofile von *PPAR-gamma* und *HMGA2* von vier Typ II-Diabetikern, erstellt aus der Abweichung der Mittelwerte der Expression des entsprechenden Gens vom jeweiligen Mittelwert der Expression des Gesamtprobandenkollektivs, sind charakteristisch (Fig. 10). Die dargestellten Gen-Expressionsprofile weisen eine positive Abweichung der *HMGA2*-Expression und eine negative bzw. äußerst niedrige *PPAR*-gamma-Expression vom Mittelwert des Gesamtprobandenkollektivs auf. Diese Genexpressionsprofile deuten auf einen hohen Anteil von Präadipozyten und einen niedrigen Anteil an reifen insulin-sensitiven Adipozyten hin. Der BMI-Status der Diabetiker reichte von normalgewichtig (Diabetiker 1: BMI 23,4 kg/m²) über übergewichtig (Diabetiker 2: BMI 26,5 kg/m²) bis zu Adipositas Grad I (Diabetiker 3: BMI 31,5 kg/m²) und Adipositas Grad II (Diabetiker4: BMI 36,0 kg/m²).

Diese Ergebnisse zeigen, dass der BMI als Marker für eine bestehende bzw. sich entwickelnde Diabetes mellitus Typ II Erkrankung nur ein schwacher Indikator ist. Im Gegensatz dazu zeigt die Genexpressionsprofilanalyse von *PPAR-gamma* und *HMGA2* ein charakteristisches Profil bei Individuen, die an Diabetes mellitus Typ II erkrankt sind.

## Patentansprüche

1. Verfahren zur Prognose einer Adipositas und/oder der Prognose und/oder Diagnose von Diabetes umfassend die Schritte:
a) Bereitstellen einer Probe aus Fettgewebe aus einem Spender,
b) Bestimmen des Genexpressionslevels der Gene für *HMGA2* und *PPAR-gamma,* in der Probe und
c) Eingruppieren des Spenders der Probe in eine von wenigstens vier Risikogruppen unter Berücksichtigung der Genexpressionslevels der Gene für *HMGA2* und *PPAR-gamma* in der Probe und des Körperfettanteils, insbesondere des BMI des Spenders, wobei die Einordnung in eine der Gruppen erfolgt ausgewählt aus der Gruppe bestehend aus
a) erhöhtes relatives Genexpressionslevel für *HMGA2,* verringertes relatives Genexpressionslevel für *PPAR-gamma* und erhöhter Körperfettanteil insbesondere mittlerer BMI ≥ 25,
b) verringertes relatives Genexpressionslevel für *HMGA2,* verringertes relatives Genexpressionslevel für *PPAR-gamma* und erhöhter Körperfettanteil insbesondere mittlerer BMI ≥ 25,
c) verringertes relatives Genexpressionslevel für *HMGA2,* verringertes Genexpressionslevel für *PPAR-gamma* und nicht erhöhter Körperfettanteil insbesondere mittlerer BMI < 25,
d) verringertes relatives Genexpressionslevel für *HMGA2,* erhöhtes relatives Genexpressionslevel für *PPAR-gamma* und nicht erhöhter Körperfettanteil insbesondere mittlerer BMI < 25,
wobei das Bereitstellen in Schritt a) so erfolgt, dass das Verfahren kein Diagnostizierverfahren oder chirurgische Behandlung ist, das/die am menschlichen oder tierischen Körper vorgenommen wird.

2. Verfahren nach Anspruch 1, wobei der Spender ein Mensch ist.

3. Verfahren nach Anspruch 1 oder 2, wobei die Krankheit Diabetes mellitus Typ II ist.

4. Verfahren nach einem der vorangehenden Ansprüche, wobei die Probe durch Punktion des subkutanen abdominalen Fettgewebes gewonnen wurde.

5. Verfahren nach einem der vorangehenden Ansprüche, wobei die Probenmasse ≤ 50 mg, bevorzugt ≤ 20 mg und besonders bevorzugt ≤ 5 mg ist.

6. Verfahren nach einem der vorangehenden Ansprüche, wobei die Bestimmung des Genexpressionslevels auf mRNA-Ebene erfolgt.

7. Verfahren nach Anspruch 6, wobei vor Bestimmung des Genexpressionslevels eine Prä-Amplifizierung von cDNA erfolgt.

8. Verfahren nach einem der vorangehenden Ansprüche, wobei die Eingruppierung in Schritt c) unter Verwendung des multivariaten Modells der selbstorganisierenden Karten nach Kohonen erfolgt.

9. Verfahren nach einem der vorangehenden Ansprüche, wobei die Eingruppierung in eine von wenigstens fünf oder wenigstens sechs Risikogruppen erfolgt unter Berücksichtigung der Gruppen
e) erhöhtes relatives Genexpressionslevel für *HMGA2* verringertes relatives Genexpressionslevel für *PPAR-gamma* und nicht erhöhter Körperfettanteil insbesondere mittlerer BMI < 25, und/oder
f) verringertes relatives Genexpressionslevel für *HMGA2,* erhöhtes relatives Genexpressionslevel für *PPAR-gamma* und erhöhter Körperfettanteil insbesondere mittlerer BMI ≥ 25.

10. Verfahren nach einem der vorangehenden Ansprüche, wobei der Genexpressions-level auf mRNA-Ebene relativ zu dem Genexpressionslevel eines Housekeeping-Gens gemessen wird.

## Claims

1. Method for the prognosis of obesity and/or the prognosis and/or diagnosis of diabetes, comprising the steps:
a) providing a sample of adipose tissue from a donor,
b) determining the gene expression level of the genes for *HMGA2* and *PPAR-gamma* in the sample and
c) classing the donor of the sample into one of at least four risk groups taking into account the gene expression levels of the genes for *HMGA2* and *PPAR-gamma* in the sample and the body fat percentage, in particular the BMI of the donor, wherein the classification is done into one of the groups selected from the group consisting of
a) elevated relative gene expression level for *HMGA2,* reduced relative gene expression level for *PPAR-gamma* and elevated body fat percentage, in particular average BMI ≥ 25,
b) reduced relative gene expression level for *HMGA2,* reduced relative gene expression level for *PPAR-gamma* and elevated body fat percentage, in particular average BMI ≥ 25,
c) reduced relative gene expression level for *HMGA2,* reduced gene expression level for *PPAR-gamma* and non-elevated body fat percentage, in particular average BMI < 25,
d) reduced relative gene expression level for *HMGA2,* elevated relative gene expression level for *PPAR-gamma* and non-elevated body fat percentage, in particular average BMI < 25,
wherein the provision in step a) is done such that the method is not a diagnostic method performed on the human or animal body.

2. Method according to Claim 1, wherein the donor is a human being.

3. Method according to Claim 1 or 2, wherein the disease is type II diabetes mellitus.

4. Method according to any of the preceding claims, wherein the sample was obtained by puncture of subcutaneous abdominal adipose tissue.

5. Method according to any of the preceding claims, wherein the sample mass is ≤ 50 mg, preferably ≤ 20 mg and particularly preferably ≤ 5 mg.

6. Method according to any of the preceding claims, wherein the determination of the gene expression level is done at the mRNA level.

7. Method according to Claim 6, wherein pre-amplification of cDNA is done before determination of the gene expression level.

8. Method according to any of the preceding claims, wherein the classing in step c) is done using the multivariate model of self-organizing maps according to Kohonen.

9. Method according to any of the preceding claims, wherein the classing is done into one of at least five or at least six risk groups taking into account the groups
e) elevated relative gene expression level for *HMGA2,* reduced relative gene expression level for *PPAR-gamma* and non-elevated body fat percentage, in particular average BMI < 25, and/or
f) reduced relative gene expression level for *HMGA2,* elevated relative gene expression level for *PPAR-gamma* and elevated body fat percentage, in particular average BMI ≥ 25.

10. Method according to any of the preceding claims, wherein the gene expression level is measured at the mRNA level relative to the gene expression level of a housekeeping gene.

## Revendications

1. Méthode pour effectuer le pronostic d'une obésité et/ou le pronostic et/ou le diagnostic du diabète comprenant les étapes :
a) de fourniture d'un échantillon de tissu adipeux d'un donneur,
b) de détermination du niveau d'expression génique des gènes pour *HMGA2* et *PPAR-gamma,* dans l'échantillon et
c) de classification du donneur de l'échantillon dans un des au moins quatre groupes à risque en tenant compte du niveau d'expression génique des gènes pour *HMGA2* et *PPAR-gamma* dans l'échantillon et de la part en graisse corporelle, en particulier de l'IMC du donneur, dans lequel le classement est effectué dans un des groupes sélectionnés à partir du groupe se composant de
a) niveau d'expression génique relatif élevé pour *HMGA2,* niveau d'expression génique relatif réduit pour *PPAR-gamma* et part en graisse corporelle élevée en particulier IMC médian ≥ 25,
b) niveau d'expression génique relatif réduit pour *HMGA2,* niveau d'expression génique relatif réduit pour *PPAR-gamma* et part en graisse corporelle élevée, en particulier IMC médian ≥ 25,
c) niveau d'expression génique relatif réduit pour *HMGA2,* niveau d'expression génique réduit pour *PPAR-gamma* et part en graisse corporelle non élevée, en particulier IMC médian < 25,
d) niveau d'expression génique relatif réduit pour *HMGA2,* niveau d'expression génique relatif élevé pour *PPAR-gamma* et part en graisse corporelle non élevée, en particulier IMC médian < 25,
dans laquelle la fourniture à l'étape a) est effectuée de sorte que la méthode ne soit pas une méthode de diagnostic qui est entreprise sur le corps humain ou animal.

2. Méthode selon la revendication 1, dans laquelle le donneur est un humain.

3. Méthode selon la revendication 1 ou 2, dans laquelle la maladie est un diabète sucré de type II.

4. Méthode selon l'une des revendications précédentes, dans laquelle l'échantillon a été obtenu par ponction du tissu adipeux abdominal sous-cutané.

5. Méthode selon l'une des revendications précédentes, dans laquelle la masse d'échantillon est ≤ 50 mg, de préférence ≤ 20 mg et de manière particulièrement préférée ≤ 5 mg.

6. Méthode selon l'une des revendications précédentes, dans laquelle la détermination du niveau d'expression génique est effectuée sur le plan de l'ARNm.

7. Méthode selon la revendication 6, dans laquelle une préamplification de l'ADNc est effectuée avant la détermination du niveau d'expression génique.

8. Méthode selon l'une des revendications précédentes, dans laquelle la classification à l'étape c) est effectuée en utilisant le modèle à variables multiples des cartes auto-organisatrices selon Kohonen.

9. Méthode selon l'une des revendications précédentes, dans laquelle la classification est effectuée dans un d'au moins cinq ou au moins six groupes à risques en tenant compte des groupes
e) niveau d'expression génique relatif élevé pour *HMGA2,* niveau d'expression génique relatif réduit pour *PPAR-gamma* et part en graisse corporelle non élevée en particulier IMC médian < 25, et/ou
f) niveau d'expression génique relatif réduit pour *HMGA2,* niveau d'expression génique relatif élevé pour *PPAR-gamma* et part en graisse corporelle élevée en particulier IMC médian ≥ 25.

10. Méthode selon l'une des revendications précédentes, dans laquelle le niveau d'expression génique est mesuré sur le plan de l'ARNm par rapport au niveau d'expression génique d'un gène domestique.
